Europäisches Patentamt

European Patent Office

Office européen des brevets

(19)

(11) Numéro de publication : **0 386 619 B1**

(12)

# FASCICULE DE BREVET EUROPEEN

(45) Date de publication du fascicule du brevet :
**01.09.93 Bulletin 93/35**

(51) Int. Cl.⁵ : **A61B 5/021, A61B 8/04**

(21) Numéro de dépôt : **90103964.4**

(22) Date de dépôt : **01.03.90**

(54) **Procédé et dispositif pour déterminer la pression artérielle de manière non invasive.**

(30) Priorité : **08.03.89 CH 856/89**
**13.03.89 FR 8903365**

(43) Date de publication de la demande :
**12.09.90 Bulletin 90/37**

(45) Mention de la délivrance du brevet :
**01.09.93 Bulletin 93/35**

(84) Etats contractants désignés :
**DE DK ES GB IT NL SE**

(56) Documents cités :
**US-A- 4 425 920**
**International Conference on Biomedical**
**Transducers vol. 1, 03 novembre 1975,**
**Paris(FR) pages 271 - 276; J.P.Atherton:**
**"Mesure non-invasive de la tension artérielle**
**humaine"**

(56) Documents cités :
**MEDICAL AND BIOLOGICAL ENGINEERING**
**AND COMPUTING. vol. 25, no. 2, mars 1987,**
**STEVENAGE GB pages 189 - 194; M.Eriksen:**
**"Noninvasive measurement of arterial diame-**
**ters in humans using ultrasound echoes with**
**prefiltered waveforms"**

(73) Titulaire : **ASULAB S.A.**
**Faubourg du Lac 6**
**CH-2501 Bienne (CH)**

(72) Inventeur : **Meister, Jean-Jacques**
**Chemin de l'Eglise 10**
**CH-1066 Epalinges (CH)**
Inventeur : **Tardy, Yanik**
**Rue du Crêt 4bis**
**CH-1006 Lausanne (CH)**

(74) Mandataire : **de Raemy, Jacques et al**
**ICB Ingénieurs Conseils en Brevets SA**
**Passage Max. Meuron 6**
**CH-2001 Neuchâtel (CH)**

EP 0 386 619 B1

**Description**

La présente invention est relative à un procédé pour déterminer en chaque instant la pression sanguine p(t) dans une artère en un point donné de son parcours et à un dispositif pour la mise en oeuvre de ce procédé.

On sait que la compliance artérielle, qui est la variation de section de l'artère pour une variation correspondante de la pression, est le reflet du comportement élastique de l'artère. Cette compliance est considérée comme indispensable à la bonne connaissance de la physiologie, la physiopathologie et la thérapeutique du système artériel. Cette compliance est fonction de la pression artérielle et l'on a donc besoin, pour l'établir, de la relation instantanée qui existe entre la pression et le diamètre en un point donné de l'artère.

Des propositions pour mesurer la relation pression-diamètre ont déjà été faites, par exemple dans l'étude présentée aux pages 789 à 793 de la revue Arch. Mal. Coeur, no 6, 1987, où le comportement visco-élastique de l'aorte chez le chien conscient est analysé. La réponse visco-élastique de l'aorte à l'administration d'hormones est observée dans l'étude citée en analysant la relation pression-diamètre aortique. Cette relation est établie au moyen d'un microcapteur de pression, calibrable in situ introduit à travers l'artère humérale gauche et placé dans la lumière de l'aorte descendante et de deux cristaux piézoélectriques de 4 mm de diamètre diamétralement fixés dans l'adventice de l'aorte descendante proximale.

Les moyens qui viennent d'être évoqués ont un caractère invasif, c'est-à-dire touchent à l'intégrité des organes sur lesquels ils interviennent. Or, sur le corps humain, on préfère faire appel à des capteurs permettant des mesures non invasives, ces capteurs restant disposés à la superficie de l'artère à mesurer, sans pénétration aucune dans les tissus environnants.

Des capteurs non invasifs permettant la mesure continue de la pression sanguine sont connus. Il s'agit notamment du photopléthysmographe commercialisé par la Société Ohmeda, 3030 Airco Drive, Madison, Wisconsin, USA et portant la marque déposée "finapres" (pour finger arterial pressure). Comme indiqué, l'appareil mesure la pression sanguine à l'extrémité d'un doigt selon la manière décrite dans l'article "Effects of peripheral vasoconstriction on the measurement of blood pressure in a finger" dans la revue Cardiovascular Research, 1985, 19, 139-145.

Des capteurs non invasifs permettant la mesure du diamètre artériel sont également connus. Il s'agit notamment de l'appareil utilisé dans le document US-A-4,370,985 qui permet la mesure du diamètre de l'artère par l'envoi d'une onde ultrasonique sur l'artère et la mesure des échos renvoyés par les parois de l'artère. Cette mesure de diamètre peut s'effectuer sur des artères superficielles, par exemple l'artère humérale ou l'artère radiale.

De la brève description des capteurs connus actuellement qui vient d'être donnée ci-dessus, il ressort qu'il n'est pas possible de mesurer non invasivement la pression dans toute artère autre que celle du doigt et le diamètre de ladite artère au même endroit de sorte que la relation ou courbe pression-diamètre présente une hystérèse systématique. Cela est dû au fait que, la vitesse de propagation de l'onde de pression étant finie, les variations de pression mesurées en aval accusent un certain retard par rapport aux variations du diamètre correspondant. Ce retard est bien sûr plus important lorsque la distance qui sépare les deux sites de mesure augmente.

Pour obvier à cet inconvénient, le procédé et le dispositif de la présente invention proposent de déterminer la pression sanguine à l'endroit même où est mesuré le diamètre de l'artère et cela en utilisant seulement deux capteurs de diamètre de l'artère placés sur la peau au voisinage l'un de l'autre, ces capteurs permettant de déduire des valeurs qu'ils livrent, la valeur de la pression sanguine. Pour cela le procédé mis en oeuvre dans la présente invention est caractérisé par le fait qu'il comporte la succession des étapes suivantes :

a) on mesure de façon non invasive et simultanément pendant au moins un cycle cardiaque un premier diamètre $D_1(t)$ de l'artère en un premier endroit et un second diamètre $D_2(t)$ de l'artère en un second endroit, lesdits premier et second endroits étant séparés par une distance $\Delta x$,

b) on mémorise en des instants successifs du cycle cardiaque des couples de valeurs comportant chacun une valeur $D_1(t)$ dudit premier diamètre et une valeur $D_2(t)$ dudit second diamètre,

c) pour les couples de diamètre ainsi mémorisés, on cherche la valeur du diamètre $D_2(t + \Delta t)$ telle que $D_2(t + \Delta t) = D_1(t)$ pour déterminer ainsi le retard temporel $\Delta t(D)$ entre les mesures de diamètres de chacun des couples,

d) on calcule, à partir dudit retard $\Delta t(D)$ et de chaque valeur du premier diamètre $D_1(t)$ initialement mémorisée à l'étape b), la vitesse de propagation $c(D)$ de l'onde de pression engendrée par la fonction cardiaque en tenant compte de ladite distance $\Delta x$ au moyen de la relation $c(D) = \Delta x/\Delta t(D)$,

e) on choisit une relation mathématique $D(p) = D(p,\alpha,\beta,\gamma,...)$ rendant compte du comportement de l'artère,

f) on détermine une expression algébrique $c(D) = c(D,\alpha,\beta,\gamma,...)$ exprimant la vitesse théorique de propagation de l'onde de pression en fonction du diamètre à partir de la relation mathématique précédemment choisie à l'étape e),

g) on calcule par une méthode mathématique d'ajustement sur les valeurs de vitesse de propagation obtenues à l'étape d), les paramètres

$\alpha,\beta,\gamma,...$ de l'expression algébrique précédemment obtenue à l'étape f, et

h) on calcule la pression sanguine p(t), pour chaque valeur de premiers diamètres $D_1(t)$ précédemment mémorisée à l'étape b), en remplaçant par leurs valeurs les paramètres $\alpha,\beta,\gamma,...$ de la relation mathématique $D(p) = D(p,\alpha,\beta,\gamma,...)$ choisie à l'étape e).

C'est aussi un but de la présente invention de proposer un dispositif pour mettre en oeuvre le procédé ci-dessus défini et cela à l'aide de deux capteurs de diamètre, d'un calculateur et d'un écran de visualisation.

L'invention va être comprise maintenant à la lumière de la description suivante donnée à titre d'exemple et en s'aidant du dessin dans lequel :

- la figure 1 est une vue schématique du dispositif de mesure selon l'invention montrant une artère du bras, deux capteurs de diamètre étant disposés sur la peau à proximité de ladite artère et un calculateur équipé d'un écran de visualisation.
- la figure 2 est un diagramme montrant le signal $D_1(t)$ capté par le premier capteur de diamètre schématisé à la figure 1,
- la figure 3 est un diagramme montrant le signal $D_2(t)$ capté par le second capteur de diamètre schématisé à la figure 1,
- la figure 4 est un diagramme où les signaux des figures 2 et 3 ont été superposés avec une échelle des temps commune.
- la figure 5 est un diagramme montrant la vitesse de propagation de l'onde de pression dans l'artère en fonction du diamètre de l'artère,
- la figure 6 est un diagramme montrant une courbe résultant d'un ajustement effectué sur l'ensemble des points du diagramme de la figure 5,
- la figure 7 est un diagramme montrant la pression artérielle en fonction du temps, diagramme découlant de celui de la figure 6,
- la figure 8 est un diagramme représentant la compliance de l'artère en fonction de la pression,
- la figure 9 est un diagramme représentant la vitesse de propagation de l'onde de pression en fonction de la pression, et
- la figure 10 est un organigramme montrant comment s'enchaînent les diverses étapes du procédé selon l'invention.

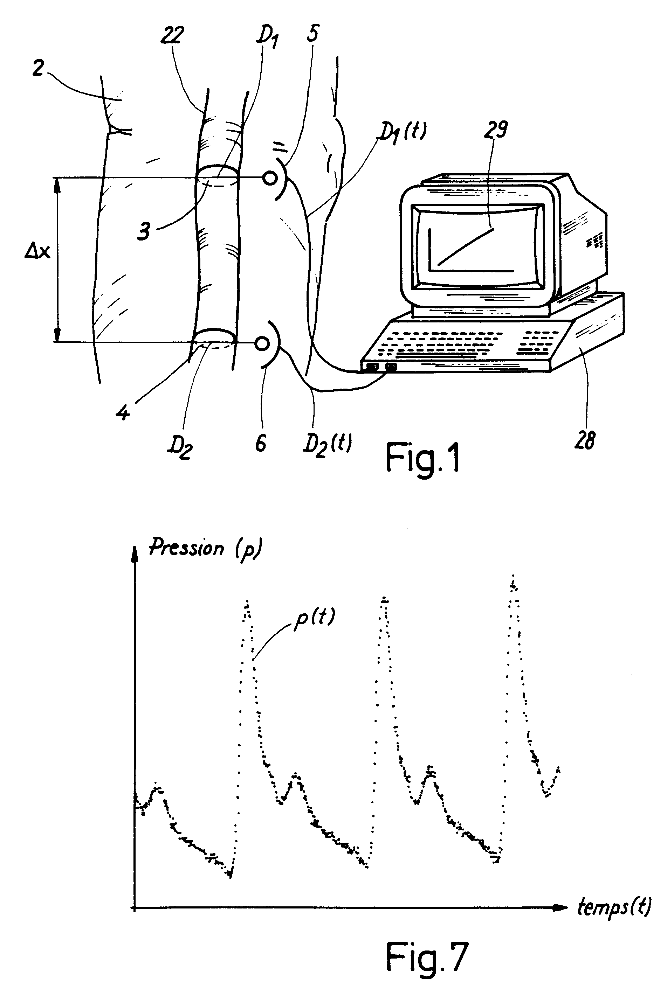

En figure 1, on a présenté un tronçon d'artère superficielle 22 enfermé dans un bras 2. Cette artère peut être, par exemple, l'artère humérale. En un premier endroit 3 de cette artère on mesure le diamètre $D_1(t)$ et en un second endroit 4, le diamètre $D_2(t)$. Les endroits 3 et 4 sont séparés par une distance $\Delta x$. Les capteurs utilisés pour cette mesure sont disposés sur le bras du patient et symbolisés sur la figure par les références 5 et 6. Il s'agit donc de mesures non invasives n'exigeant aucune introduction dans le bras et les capteurs utilisés à cet effet sont du type à émission ultrasonore captant les échos sur les parois de l'artère, comme on l'a dit plus haut. Les signaux $D_1(t)$ et $D_2(t)$ issus respectivement des capteurs 5 et 6 sont envoyés à un calculateur 28 pour traitement. Le calculateur est complété par un écran de visualisation 29. Les mesures ont lieu pendant au moins un cycle cardiaque.

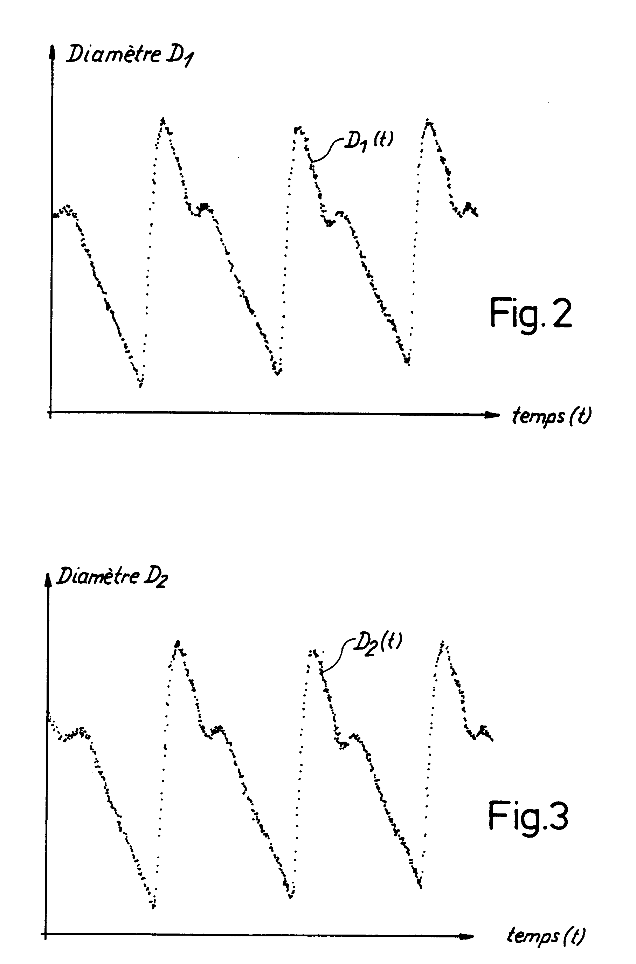

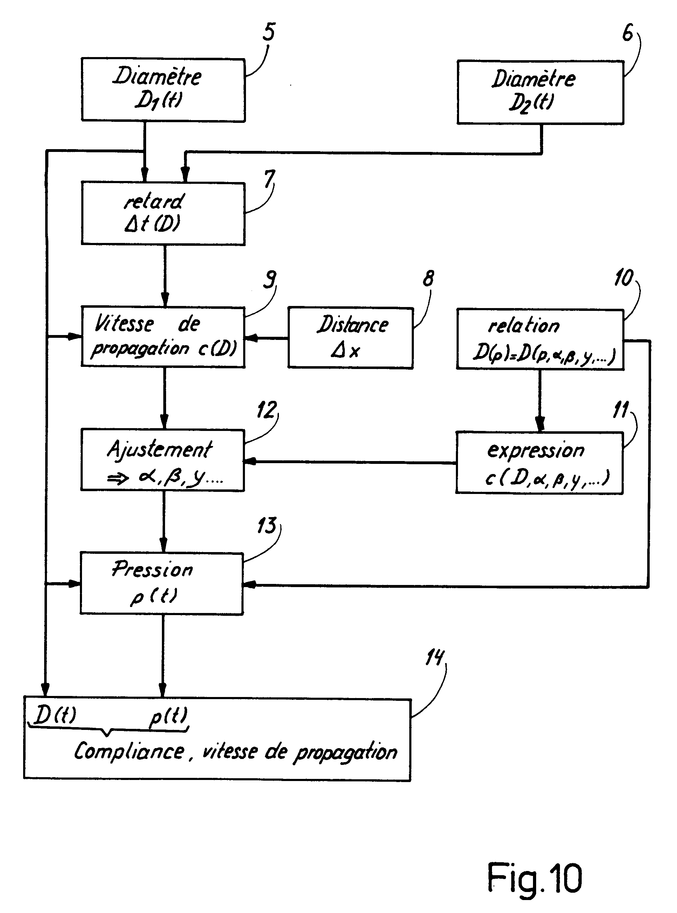

A l'aide de l'arrangement qui vient d'être décrit, le procédé selon l'invention pour déterminer la pression sanguine p(t) d'une artère en un point donné de son parcours va être expliqué maintenant à l'aide de l'organigramme de la figure 10 et des divers diagrammes montrés en figures 2 à 7.

La figure 2 montre le signal $D_1(t)$ issu du capteur 5. Le diagramme montre la variation du diamètre $D_1$ de l'artère en fonction du temps sur environ trois cycles cardiaques. La figure 3 montre le signal $D_2(t)$ issu du capteur 6. De la même façon, ce diagramme montre la variation du diamètre $D_2$ de l'artère en fonction du temps t sur environ trois cycles cardiaques. Ainsi pour une même valeur de temps $t_1$, par exemple, on recueille un couple de valeurs de diamètres $D_1(t_1)$ et $D_2(t_1)$ et ainsi de suite pour les autres valeurs du temps.

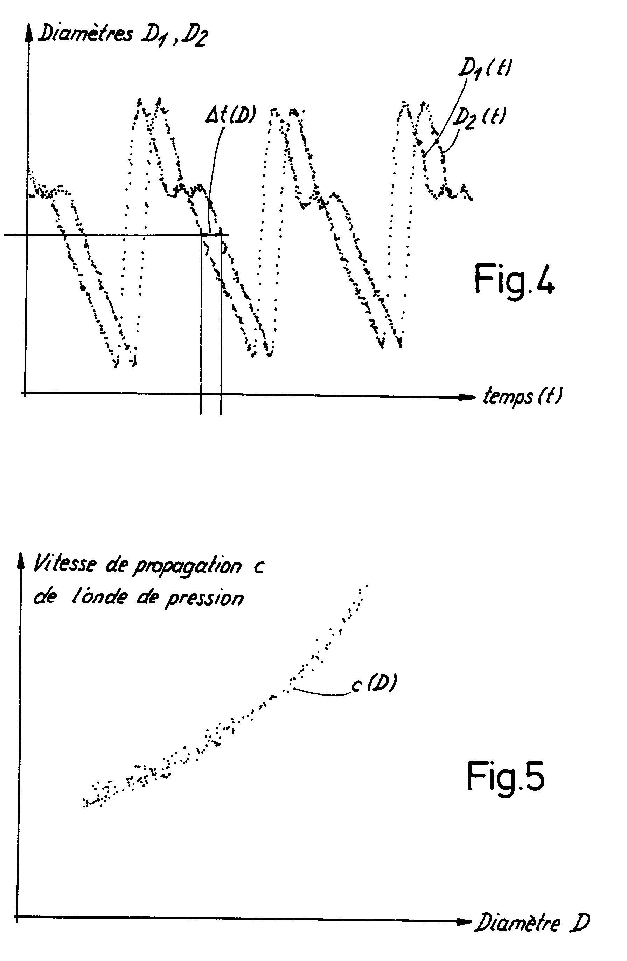

On mémorise ensuite (blocs 5 et 6 de l'organigramme de la figure 10), dans le calculateur 28, en des instants successifs du cycle cardiaque, les couples de valeurs ainsi mesurés, et on cherche la valeur du diamètre $D_2(t + \Delta t)$ telle que $D_2(t + \Delta t) = D_1(t)$. L'opération est figurée dans le diagramme de la figure 4 qui est une superposition des diagrammes des figures 2 et 3. Cette opération permet ainsi de déterminer le retard temporel $\Delta t(D)$ existant entre les mesures de diamètres de chacun des couples mémorisés. Ce retard, qui est une fonction du diamètre D de l'artère est calculé et stocké dans le bloc 7 de l'organigramme de la figure 10.

On calcule maintenant, à partir du retard $\Delta t (D)$ et de chaque valeur du premier diamètre $D_1(t)$ mesurée au bloc 5 de l'organigramme, la vitesse de propagation c(D) de l'onde de pression engendrée par la fonction cardiaque, au moyen de la relation $c(D) = \Delta x/\Delta t(D)$, $\Delta x$ étant la distance séparant les capteurs 5 et 6. Ce calcul est symbolisé par le bloc 9 de l'organigramme de la figure 10 et la distance $\Delta x$ est stockée dans le bloc 8 montré au même organigramme. On notera ici que la distance $\Delta x$ peut être choisie de l'ordre de 2 cm ce qui permet de différencier nettement les courbes $D_1(t)$ et $D_2(t)$. Avec cet ordre de grandeur, le décalage en temps est de l'ordre de la milliseconde. La vitesse de propagation de l'onde de pression c(D) est représentée graphiquement à la figure 5.

On va choisir maintenant une relation mathématique $D(p) = D(p,\alpha,\beta,\gamma,...)$ tenant compte du comportement de l'artère. Cette relation, qu'on stocke dans le bloc 10 de l'organigramme de la figure 10, est donnée par l'expérience. Elle pourrait être de la forme exponentielle :

$$p = \alpha e^{\frac{\beta \pi D^2}{4}}$$

ou encore d'une forme donnée dans la revue J. Biomechanics, Vol. 17, no 6, pp. 425-435, 1984 et qui s'écrit :

$$S = \alpha[1/2 + \tan^{-1}[(p - \beta)/\gamma]/\pi]$$

où $S = \frac{\pi D^2}{4}$. On notera que dans les nombreuses relations proposées dans la littérature, le nombre de paramètres $\alpha, \beta, \gamma, ...$ est variable.

La prochaine étape du procédé selon l'invention consiste à déterminer une expression algébrique $c(D) = c(D, \alpha, \beta, \gamma, ...)$ qui exprime la vitesse théorique de propagation de l'onde de pression en fonction du diamètre à partir de la relation mathématique dont il a été question ci-dessus. Cette expression peut être obtenue de différentes manières, par exemple en utilisant l'expression

$$c(p) = \sqrt{\frac{S}{\rho} \cdot \frac{dp}{dS}}$$

pù $S = \frac{\pi D^2}{4}$, où $\rho$ est la densité du sang et où $\frac{dp}{dS}$ est la dérivée de la pression par la section. On notera que l'expression $c(p)$ est connue de l'étude de l'hémodynamique artérielle. Cette étape est symbolisée par le bloc 11 de l'organigramme de la figure 10.

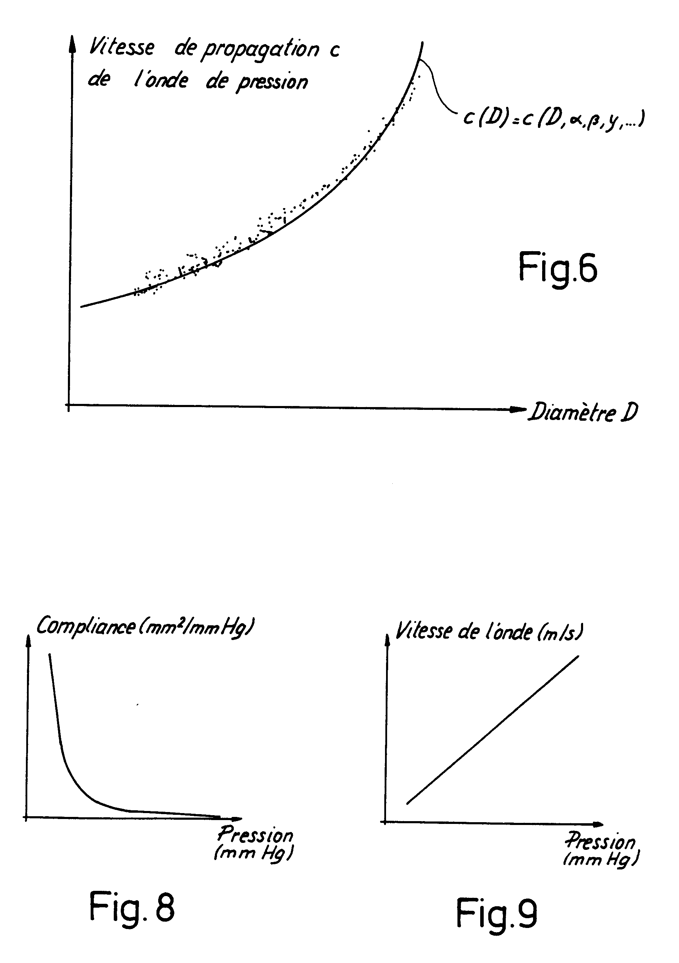

Une démarche importante du procédé selon l'invention consiste alors à utiliser les valeurs de vitesse de propagation $c(D)$ contenues dans le bloc 9 et les valeurs théoriques de vitesse de propagation $c(D, \alpha, \beta, \gamma, ...)$ pour calculer les paramètres $\alpha, \beta, \gamma, ...$ en utilisant une méthode mathématique d'ajustement ou routine connue de l'état de l'art, par exemple, la méthode des moindres carrés. D'autres méthodes sont possibles et sont décrites en détail dans l'ouvrage "Numerical Recipes" publié par "The Press Syndicate of the University of Cambridge" 1986. Très généralement, il s'agit d'un procédé mathématique standard de minimisation des écarts. Cet ajustement (ou "fit") est présent à la sortie du bloc 12 de l'organigramme de la figure 10. Le résultat de cet ajustement est montré par la courbe pleine de la figure 6.

Enfin, en remplaçant par leurs valeurs les paramètres $\alpha, \beta, \gamma, ...$ obtenus à l'étape précédente dans la relation $D(p) = D(p, \alpha, \beta, \gamma, ...)$ contenue dans le bloc 10, on peut calculer la pression sanguine $p(t)$ pour chaque valeur de premiers diamètres $D_1(t)$ obtenue précédemment à l'étape symbolisée par le bloc 5. La configuration de cette pression en fonction du temps est représentée sur le diagramme de la figure 7 et ses valeurs sont présentes au bloc 13 de l'organigramme de la figure 10.

Ainsi a-t-on réalisé le but que se proposait d'atteindre la présente invention, à savoir la détermination de la pression sanguine à chaque instant du cycle cardiaque, détermination basée sur la seule mesure du diamètre de l'artère en deux endroits distincts de son parcours.

Cette mesure peut être effectuée sur l'artère humérale comme on l'a dit plus haut. Elle pourait être effectuée à d'autres endroits de n'importe quelle artère superficielle par exemple de la jambe ou du cou.

Des valeurs de la pression $p(t)$ et du diamètre $D_1(t)$ en un endroit unique 3 de l'artère, il est aisé alors d'établir la relation pression-diamètre $D(p)$ de ladite artère. De cette relation, on peut alors obtenir la valeur de la compliance en fonction de la pression (figure 8) et de la vitesse de propagation de l'onde de pression $c(p)$ (figure 9).

On mentionnera encore que toutes les étapes du procédé selon l'invention, ainsi que les calculs qui apparaissent dans l'organigramme de la figure 11, peuvent être réalisés au moyen d'un ordinateur vendu sur le marché, par exemple au moyen de l'appareil de la marque Olivetti M28. De même, l'écran de visualisation 29 permet de faire apparaître, à la demande du praticien, n'importe quel graphique en cours ou en fin de procédé.

## Revendications

1. Procédé pour déterminer en chaque instant la pression sanguine $p(t)$ dans une artère (22) en un point donné de son parcours, caractérisé par le fait qu'il comporte la succession des étapes suivantes :

   a) on mesure de façon non invasive et simultanément pendant au moins un cycle cardiaque un premier diamètre $D_1(t)$ de l'artère en un premier endroit (3) et un second diamètre $D_2(t)$ de l'artère en un second endroit (4), lesdits premier et second endroits étant séparés par une distance $\Delta x$,

   b) on mémorise (5, 6) en des instants successifs du cycle cardiaque des couples de valeurs comportant chacun une valeur $D_1(t)$ dudit premier diamètre et une valeur $D_2(t)$ dudit second diamètre,

   c) pour les couples de diamètre ainsi mémorisés, on cherche (7) la valeur du diamètre $D_2(t + \Delta t)$ telle que $D_2(t + \Delta t) = D_1(t)$ pour déterminer ainsi le retard temporel $\Delta t(D)$ entre les mesures de diamètres de chacun de ces couples,

   d) on calcule (9), à partir dudit retard $\Delta t(D)$ et de chaque valeur du premier diamètre $D_1(t)$ initialement mémorisée à l'étape b), la vitesse de propagation $c(D)$ de l'onde de pression engendrée par la fonction cardiaque en tenant compte (8) de ladite distance $\Delta x$ au moyen de la relation $c(D) = \Delta x/\Delta t(D)$,

   e) on choisit (10) une relation mathématique $D(p) = D(p, \alpha, \beta, \gamma, ...)$ rendant compte du

comportement de l'artère,

f) on détermine (11) une expression algébrique c(D) = c(D,$\alpha$, $\beta$,$\gamma$,...) exprimant la vitesse théorique de propagation de l'onde de pression en fonction du diamètre à partir de la relation mathématique précédemment choisie à l'étape e),

g) on calcule (12) par une méthode mathématique d'ajustement sur les valeurs de vitesse de propagation obtenues à l'étape d), les paramètres $\alpha$,$\beta$,$\gamma$,... de l'expression algébrique précédemment obtenue à l'étape f), et

h) on calcule (13) la pression sanguine p(t), pour chaque valeur de premiers diamètres $D_1(t)$ précédemment mémorisée à l'étape b), en remplaçant par leurs valeurs les paramètres de la relation mathématique D(p) = D(p,$\alpha$,$\beta$,$\gamma$,...) choisie à l'étape e).

2. Procédé selon la revendication 1, caractérisé par le fait que, pour déterminer (11) l'expression algébrique c(D) = c(D,$\alpha$,$\beta$, $\gamma$,...) de l'étape f), on utilise l'expression

$$c(p) \;=\; \sqrt{\frac{S}{\rho} \cdot \frac{dp}{dS}}$$

où $S = \dfrac{\pi D^2}{4}$ et où $\rho$ est la densité du sang.

3. Dispositif pour la mise en oeuvre du procédé selon la revendication 1, caractérisé par le fait qu'il comporte des premier et second capteurs (5, 6) non invasifs pour mesurer en deux endroits (3, 4) séparés par une distance $\Delta x$, un premier et un second diamètre ($D_1$, $D_2$) d'une artère (22), un calculateur (28) pour traiter des valeurs livrés par lesdits capteurs et établir la valeur de la pression sanguine p(t) dans ladite artère et un écran de visualisation (29).

## Claims

1. Method for determining at each instant the blood pressure p(t) in an artery (22) at a given point in its course, characterized by the fact that it includes the succession of the following steps :

a) measuring non-invasively and simultaneously during at least one cardiac cycle a first diameter $D_1(t)$ of the artery at a first location (3) and a second diameter $D_2(t)$ of the artery at a second location (4), said first and second locations being separated by a distance $\Delta x$,

b) memorizing (5, 6) at successive instants of the cardiac cycle pairs of values each including a value $D_1(t)$ of said first diameter and a value $D_2(t)$ of said second diameter,

c) for the diameter pairs thus memorized, seeking (7) the value of the diameter $D_2(t+\Delta t)$ such that $D_2(t+\Delta t) = D_1(t)$ in order thus to determine the time delay $\Delta t(D)$ between the diameter measurements of each of these pairs,

d) calculating (9) on the basis of said delay $\Delta t(D)$ and of each value of the first diameter $D_1(t)$ initially memorized during step b) the propagation velocity c(D) of the pressure wave generated by the cardiac function in taking into account (8) said distance $\Delta t$ by means of the relation c(D) = $\Delta x$/$\Delta t$(D),

e) choosing a mathematical relationship D(p) = D(p, $\alpha$, $\beta$, $\gamma$, ...) which takes the behaviour of the artery into account,

f) determining (11) an algebraic expression c(D) = c(D, $\alpha$, $\beta$, $\gamma$, ...) expressing the theoretical propagation velocity of the pressure wave as a function of the diameter from the preceding mathematical relationship chosen in step e),

g) calculating (12) by a mathematical adjustment method on the propagation velocity values obtained during step d) the parameters $\alpha$, $\beta$, $\gamma$, ... from the algebraic expression previously obtained in step f), and

h) calculating (13) the blood pressure p(t) for each value of first diameters $D_1(t)$ previously memorized in step b) in replacing the parameters of the mathematical relationship d(p) = D(p, $\alpha$, $\beta$, $\gamma$, ...) chosen in step e) by their values.

2. Method according to claim 1, characterized by the fact that, in order to determine the algebraic expression c(D) = c(D, $\alpha$, $\beta$, $\gamma$, ...) of step f), the expression

$$c(p) \;=\; \sqrt{\frac{S}{\rho} \cdot \frac{dp}{dS}}$$

is employed,

where $S = \dfrac{\pi D^2}{4}$ and where $\rho$ is the blood density.

3. Device for carrying out the method according to claim 1, characterized by the fact that it includes first and second non-invasive sensors (5, 6) for measuring a first and a second diameter ($D_1$, $D_2$) of an artery (22) at two locations (3, 4) separated by a distance $\Delta x$, a calculator (28) for processing the values supplied by said sensors and establishing the value of the blood pressure p(t) in said artery and a visualization screen (29).

## Patentansprüche

1. Verfahren zum Bestimmen des Blutdruckes p(t) in einer Arterie (22) an einem gegebenen Punkt ihres Verlaufs in jedem Augenblick, dadurch gekennzeichnet, daß es die Abfolge der folgenden Schritte umfaßt:

a) man mißt in nicht invasiver Weise und gleichzeitig während mindestens eines kardialen Zyklus einen ersten Durchmesser $D_1(t)$ der Arterie an einer ersten Stelle (3) und einen zweiten Durchmesser $D_2(t)$ der Arterie an einer zweiten Stelle (4), welche ersten und zweiten Stellen durch eine Distanz $\Delta x$ getrennt sind,

b) man speichert (5, 6) an aufeinanderfolgenden Zeitpunkten des kardialen Zyklus Paare von Werten, umfassend jeweils einen Wert $D_1(t)$ des ersten Durchmessers und eines Wertes $D_2(t)$ des zweiten Durchmessers,

c) für die Paare von derart abgespeicherten Durchmessern sucht (7) man den Wert des Durchmessers $D_2(t + \Delta t)$ derart, daß $D_2(t + \Delta t)$ = $D_1(t)$, um auf diese Weise die zeitliche Verzögerung $\Delta t(D)$ zwischen den Messungen der Durchmesser jedes dieser Paare zu bestimmen,

d) man berechnet (9) ausgehend von der Verzögerung $\Delta t(D)$ und von jedem Wert des ersten Durchmessers $D_1(t)$, der anfänglich in Schritt b) abgespeichert worden ist, die Ausbreitungsgeschwindigkeit c(D) der Druckwelle, erzeugt durch die kardiale Funktion unter Berücksichtigung (8) der Distanz $\Delta x$ mittels der Beziehung c(D) = $\Delta x / \Delta t(D)$,

e) man wählt (10) eine mathematische Beziehung D(p) = D(p, $\alpha$, $\beta$, $\gamma$ ,....) unter Rechnungslegung des Verhaltens der Arterie,

f) man bestimmt (11) einen algebraischen Ausdruck c(D) = c(D, $\alpha$ , $\beta$, $\gamma$ ,...) unter Ausdrücken der theoretischen Ausbreitungsgeschwindigkeit der Druckwelle in Funktion des Durchmessers, ausgehend von der mathematischen Beziehung, die vorstehend in Schritt (e) gewählt wurde,

g) man berechnet (12) mittels einer mathematischen Anpaßmethode auf die Werte der in Schritt d) erhaltenen Ausbreitungsgeschwindigkeit die Parameter $\alpha$, $\beta$, $\gamma$ ,... des vorstehend in Schritt f) erhaltenen algebraischen Ausdrucks und

h) man berechnet (13) den Blutdruck p(t) für jeden Wert der ersten Durchmesser $D_1(t)$, die vorangehend in Schritt (b) abgespeichert worden waren, unter Ersatz durch ihre Werte der Parameter der mathematischen Beziehung D(p) = D(p, $\alpha$, $\beta$, $\gamma$ ,...), gewählt in Schritt e).

2. Verfahren nach Anspruch 1, dadurch gekennzeichnet, daß zum Bestimmen (11) des algebraischen Ausdrucks c(D) = c(D, $\alpha$, $\beta$, $\gamma$,...) des Schrittes (f) der Ausdruck verwendet wird

$$c(p) = \sqrt{\frac{S \cdot dp}{\rho \cdot dS}}$$

worin $S = \dfrac{\pi D^2}{4}$ und worin $\rho$ die Blutdichte sind.

3. Vorrichtung für die Durchführung des Verfahrens nach Anspruch 1, dadurch gekennzeichnet, daß sie erste und zweite nicht invasive Fühler (5, 6) zum Messen, an zwei durch eine Distanz $\Delta x$ getrennten Stellen (3, 4), eines ersten und eines zweiten Durchmessers ($D_1$, $D_2$) einer Arterie (22), einen Rechner (28) zum Verarbeiten der von den Fühlern gelieferten Werte und zum Etablieren des Blutdruckes p(t) in der Arterie und einen Visualisierungsbildschirm (29) umfaßt.

Fig.1

Fig.7

Diamètre $D_1$

$D_1(t)$

temps (t)

Fig. 2

Diamètre $D_2$

$D_2(t)$

temps (t)

Fig. 3

Diamètres $D_1$, $D_2$

$D_1(t)$

$D_2(t)$

$\Delta t(D)$

Fig.4

temps (t)

Vitesse de propagation c
de l'onde de pression

c (D)

Fig.5

Diamètre D

Vitesse de propagation c
de l'onde de pression

$c(D) = c(D, \alpha, \beta, y, ...)$

Fig.6

Diamètre D

Compliance (mm²/mm Hg)

Pression
(mm Hg)

Fig. 8

Vitesse de l'onde (m/s)

Pression
(mm Hg)

Fig.9

10

Fig.10